# Europäisches Patentamt
## European Patent Office
## Office européen des brevets

(11) Veröffentlichungsnummer: **0 149 430**
**A1**

(12) # EUROPÄISCHE PATENTANMELDUNG

(21) Anmeldenummer: 84810636.5

(22) Anmeldetag: 18.12.84

(51) Int. Cl.⁴: **C 12 N 15/00**
**C 12 N 7/00**

(30) Priorität: 04.01.84 CH 16/84
05.01.84 CH 31/84

(43) Veröffentlichungstag der Anmeldung:
24.07.85 Patentblatt 85/30

(84) Benannte Vertragsstaaten:
AT BE CH DE FR GB IT LI LU NL SE

(71) Anmelder: CIBA-GEIGY AG
Postfach
CH-4002 Basel(CH)

(72) Erfinder: Brisson, Normand, Dr. University of Montreal
Department of Biochemistry C.p. 6128
Succursale A Montreal H3C 3JF(CA)

(72) Erfinder: Dixon, Linda Katleen, Dr.
42 Alpha Road
Chobham Surrey(GB)

(72) Erfinder: Hohn, Thomas, Dr.
Talmattweg 11
CH-4103 Bottmingen(CH)

(72) Erfinder: Gronenborn, Bruno, Dr.
Im Broch
D-5010 Bergheim(DE)

(72) Erfinder: Paszkowski, Jerzy, Dr.
Oberdorfstrasse 5
CH-4125 Riehen(CH)

(72) Erfinder: Penswick, John Robert, Dr.
14, Christopher Court
Malbrook Road Norwich NR5 8QZ(GB)

(72) Erfinder: Potrykus, Ingo, Dr.
Im Stigler 54
CH-4312 Magden(CH)

(54) Verfahren zur Herstellung chimärer DNS.

(57) Verfahren zur Herstellung chimärer DNS, welche aus Virus-DNS von einem Pflanzenvirus und einem in pflanzlichem Material zur Expression gelangenden Fremdgen besteht, wobei ein in pflanzlichem Material zur Expression gelangendes Fremdgen in die DNS eines Pflanzenvirus eingesetzt wird.

./...

Croydon Printing Company Ltd.

Herstellung der chimären Plasmide

*Fig. 3*

N = Basenpaar
(NNN) = Codon

CIBA-GEIGY AG                                    5-14726/1+2/+
Basel (Schweiz)

## Verfahren zur Herstellung chimärer DNS

Die vorliegende Erfindung betrifft ein Verfahren zur Herstellung chimärer DNS, welche aus Virus-DNS von einem Pflanzenvirus und einem in pflanzlichem Material zur Expression gelangenden Fremdgen besteht.

Durch die Einführung neuer genetischer Information in pflanzliches Material können Pflanzen mit neuen oder verbesserten Eigenschaften erzeugt werden.

Angesichts des raschen Anstiegs der Weltbevölkerung gehört eine Produktion von Nutzpflanzen oder pflanzlichen Inhaltsstoffen, vorzugsweise auf dem Gebiet der Nahrungs- und Heilmittel, zu den dringlichsten Aufgaben der biologischen Forschung. In diesem Zusammenhang sind wesentliche Aspekte beispielsweise eine Erhöhung der Resistenz von Nutzpflanzen gegen Krankheiten und Schädlinge, welche durch Induktion oder vermehrte Bildung von Schutzstoffen oder Toxinen in der Pflanze herbeigeführt werden kann, eine gesteigerte Resistenz gegen Pflanzenschutzmittel wie Insektizide, Herbizide, Fungizide und Bakterizide, und eine günstige Veränderung des Nährstoffgehalts von Pflanzen, welche durch Induktion zur Bildung wertvoller Proteine oder auf andere Weise herbeigeführt werden kann. Ein besonderer Aspekt ist die Anregung pflanzlichen Materials zur Erzeugung pflanzenfremder, d.h. in den originären Pflanzen nicht gebildeter Substanzen. Eine entsprechende Beeinflussung pflanzlichen Materials kann beispielsweise durch Einbringung neuer DNS, wobei es sich um chimäre DNS handeln kann, erfolgen, wobei das pflanzliche Material in Form von Zellkulturen oder Vermehrungsgut von Nutzpflanzen oder als ganze Pflanzen vorliegen kann.

Die vorliegende Erfindung betrifft ein Verfahren zur Herstellung chimärer DNS, welches darin besteht, dass man in die DNS eines Pflanzenvirus ein in pflanzlichem Material zur Expression gelangendes Fremdgen einsetzt.

Die Erfindung betrifft ferner auch die Verwendung der chimären DNS zur Infizierung pflanzlichen Materials, wobei als Vektor ein die chimäre DNS enthaltendes Pflanzenvirus eingesetzt wird.

Weitere Aspekte der vorliegenden Erfindung betreffen Cauliflower-Mosaik-Virus, welches die chimäre DNS enthält, sowie pflanzliches Material, welches mit einem solchen Virus infiziert ist, ferner die Verwendung der chimären DNS zur Infizierung von pflanzlichem Material unter Einsatz eines solchen Virus als Vektor sowie neue Plasmide.

Bei der Durchführung der vorliegenden Erfindung geht man zweckmässigerweise so vor, dass man eine oder mehrere Regionen aus der Virus-DNS entfernt, wobei nur solche Regionen berücksichtigt werden, die für die Vermehrung des Virus, welches die chimäre DNS beherbergt, nicht erforderlich sind. In die verbleibende Virus-DNS wird dann ein Fremdgen eingeführt. Das Fremdgen wird an einer geeigneten, die Expression des Fremdgens und die Vermehrungsfähigkeit des Virus nicht beeinträchtigenden Region der Virus-DNS eingesetzt. Vorteilhafterweise wird das Fremdgen dort eingeführt, wo eine Virus DNS-Region entfernt worden ist (Deletionsort), wobei das eingefügte Fremdgen eine oder mehrere der entfernten Virus-DNS-Regionen ersetzt. Beim Vorliegen mehrerer Deletionsorte können auch mehrere Gene in die Virus-DNS eingesetzt werden, wobei es sich als besonders vorteilhaft erweist, wenn eines dieser Gene eine gut feststellbare Expression in den Pflanzen zeigt, welche als Kennzeichnung für das Vorliegen der chimären DNS verwendet werden kann.

Im allgemeinen erweist es sich als vorteilhaft, das Fremdgen in die Virus-DNS mittels Restriktionsenzymen, für welche in der Virus-DNS nur eine einzige Restriktionsschnittstelle vorliegt, so einzusetzen, dass die Erkennungssequenzen dieser Restriktionsenzyme das einzusetzende Fremdgen an beiden Enden flankieren.

Bevorzugterweise wird der Abstand zwischen dem Stopsignal des unmittelbar vor dem eingesetzten Fremdgen liegenden Virusgens und dem Startsignal des eingesetzten Fremdgens so kurz wie möglich gehalten.

Gemäss einer bevorzugten Ausführungsform des erfindungsgemässen Verfahrens werden die flankierenden Sequenzen des einzusetzenden Gens möglichst vollständig entfernt, was zweckmässigerweise auf enzymatischem Wege, beispielsweise mit einer geeigneten Nuklease, erfolgt.

Es erweist sich als günstig, den Abstand zwischen dem Stopsignal des eingesetzten Fremdgens und dem Startsignal des dem Fremdgen unmittelbar nachfolgenden Virusgens möglichst gering zu halten.

Es erweist sich als sehr vorteilhaft, ein Fremdgen einzuführen, dessen Grösse diejenige des entfernten Virus-DNS-Fragments oder diejenige der Summe aller entfernten Virus-DNS-Fragmente nicht wesentlich überschreitet.

Bei der Einführung einer oder mehrerer neuer Codierungsregionen im Rahmen der vorliegenden Erfindung wird das Leseraster der Virus-DNS beibehalten.

Das einzusetzende Fremdgen darf keine homologen Enden aufweisen und muss im richtigen Orientierungssinn eingesetzt werden.

Die chimäre DNS, welche ebenfalls einen Gegenstand der vorliegenden Erfindung bildet, kann unter Verwendung der als Virus-DNS-Spender dienenden und die chimäre DNS enthaltenden Pflanzenviren als Vektoren zur Infizierung von pflanzlichem Material beispielsweise von Zellkulturen oder Vermehrungsgut von Nutzpflanzen oder von ganzen Pflanzen eingesetzt werden, wobei im Rahmen der vorliegenden Erfindung das eingesetzte Fremdgen die vorhandene Fähigkeit zur Expression beibehält. Als Nutzpflanzen kommen insbesondere Vertreter der Familien Cruciferae, Leguminosae, Rosaceae, Compositae und Solanaceae in Betracht.

Als Virus-DNS-Spender und zugleich Vektoren kommen vorzugsweise DNS-Viren oder die DNS-Kopie einer Virus-RNS, vorzugsweise Caulimoviren und speziell originale oder recombinante Stämme des Cauliflower-Mosaik-Virus (CaMV, Blumenkohl-Mosaik-Virus) in Frage.

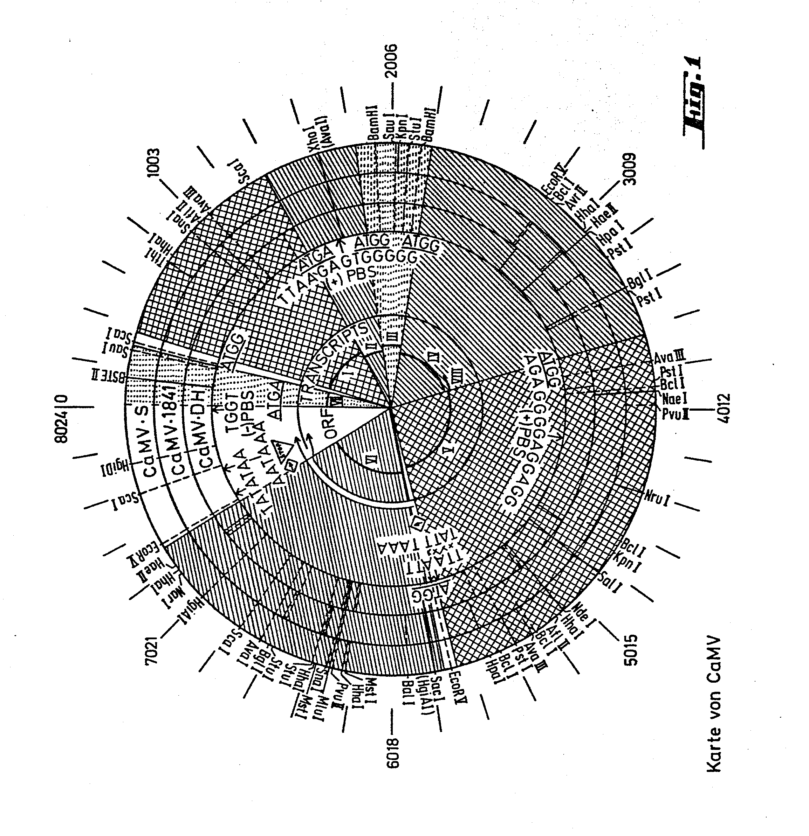

Beim Cauliflower-Mosaik-Virus lassen sich folgende DNS-Regionen (Codier-Regionen) ohne Beeinträchtigung der Vermehrungsfähigkeit des Virus entfernen: ORF II, ORF VII und Teile der intergenomen Region (Figur 1; die weissen Sektoren stellen die intergenomen Regionen dar).

Besonders erwähnenswert ist die Entfernung der Region ORF II, welche etwa 450 Basenpaare enthält, und/oder der Region ORF VII, welche 350 Basenpaare enthält, die möglich ist, ohne dass dadurch die Infektiosität der DNS bei künstlicher Inokulation beeinträchtigt wird. Es erweist sich als besonders vorteilhaft, wenn zwischen der tRNS-Bindungsstelle der Virus-DNS und der ersten, in Leserichtung folgenden ORF-Region nicht mehr als 50 bis 70 Basenpaare beibehalten werden und die nachfolgenden ORF-Regionen in möglichst kurzen Abständen folgen (die tRNS-Bindungsstelle entspricht der Position 0 in der Karte von CaMV, Figur 1).

Zweckmässigerweise wird das einzufügende Fremdgen an die Stelle des entfernten ORF II plaziert.

Das einzusetzende Gen kann von verschiedenen Organismen abstammen, beispielsweise von Bakterien. Ein besonders gut geeignetes Gen ist beispielsweise das DHFR-Gen (Dihydrofolat-Reduktase-Gen).

Die flankierenden Sequenzen des einzusetzenden Gens werden möglichst vollständig, vorzugsweise mit einem geeigneten Enzym, wie beispielsweise der Nuklease Bal 31, entfernt. Sofern auf das Startsignal ATG der Kodierungssequenz ein weiteres G folgt, was überdurchschnittlich oft der Fall ist, lässt sich mit einer Kupplersequenz, welche mit CC aufhört, genau am 5'-Ende der Kodiersequenz eine NcoI-Restriktionsstelle (CCATGG) einführen, welche sich als Kupplung verwenden lässt. Diese Sequenz dient zum Aufbau des chimären CaMV-Vektors. Die Selektionierung des gewünschten Moleküls kann mittels der NcoI-Sequenz erfolgen.

Das erfindungsgemässe Verfahren wird nachfolgend an Beispielen veranschaulicht, in denen als Virus Cauliflower-Mosaik-Virus und als Fremdgen das Plasmid-kodierte Dihydrofolat-Reduktase-Gen (DHFR-Gen) verwendet werden.

Das DHFR-Gen wird in die DNS von Cauliflower-Mosaik-Virus eingefügt, und zwar anstelle der entfernten Region ORF II.

Das einzusetzende Fremdgen, welches durch das Plasmid R67 kodiert wird, stellt das Gen für das gegen Methotrexat resistente Enzym DHFR dar und weist 234 Basenpaare auf. Die Resistenz gegen den Folsäure-Antagonisten Methotrexat (4-Amino-$N^{10}$-methylpteroylglutaminsäure) stellt ein ausgezeichnetes Kennzeichen dar, welches das Vorliegen der Expression in pflanzlichem Material leicht erkennbar macht.

Zur Prüfung der Infektionsfähigkeit der erhaltenen chimären DNS werden Pflanzen der Spezies Brassica rapa mit klonierter chimärer Virus-DNS inokuliert. Die Infektionssymptome entwickeln sich im gleichen Zeitraum wie bei Kontrollpflanzen, welche mit der originären Virus-DNS, welche dem Empfangsort für das DHFR-Gen entspricht, behandelt worden sind. Daraus kann gefolgert werden, dass das Vorliegen des DHFR-Gens die Replikation des Virus nicht nachteilig beeinflusst.

Zum Beweis, dass das DHFR-Gen im Virus-Genom verblieben ist, werden Viruspartikel aus systemisch infizierten Blättern isoliert und die daraus gewonnene DNS wird enzymatisch zerlegt. Das DNS-Fragment, welches das DHFR-Gen enthält, liegt in dem zu erwartenden molaren Anteil vor, woraus geschlossen werden kann, dass die Fremd-DNS im Virusgenom dauerhaft erhalten bleibt. Die Stabilität des eingesetzten Gens, welche in älteren Pflanzenteilen etwas weniger ausgeprägt ist als in jüngeren Pflanzenteilen, lässt sich durch Verkürzung der Entfernung zwischen dem Ende des dem Fremdgen vorgelagerten Virusgens und dem Anfang des DHFR-Gens noch verbessern. So lässt sich zeigen, dass auch nach zweimaligem aufeinanderfolgendem Transfer von Viren aus systemisch infizierten Pflanzengeweben das eingesetzte DHFR-Gen vorhanden ist und das DNS-Fragment, welches das DHFR-Gen enthält, im zu erwartenden molaren Anteil vorliegt.

Die Expression des eingesetzten Fremdgens kann mit Antikörpern gegen das Genprodukt und mit Funktionstests nachgewiesen werden und kommt in der deutlich feststellbaren Resistenz der mit der chimären DNS infizierten Pflanzen gegen Methotrexat zum Ausdruck.

Das in den nachfolgenden Beispielen beschriebene Verfahren mit dem DHRF-Gen als Fremdgen, welches in die DNS von CaMV eingeführt wird, lässt sich auch mit anderen Genen in derselben Weise oder mit Abänderungen, welche dem Fachmann geläufig sind, durchführen.

Ein Bam-HI-Fragment, wie es im nachfolgenden Beispiel 1 verwendet wird, kann nach an sich bekannten Methoden, beispielsweise durch Behandlung eines das DHFR-Gen enthaltenden Bakterienplasmids wie R67 oder R388 mit dem Restriktionsenzym Bam-HI, gewonnen werden.

Das Plasmid pUC8X, welches im nachfolgenden Beispiel 1 eingesetzt wird, lässt sich aus dem im Handel erhältlichen Plasmid pUC8 durch Einfügen eines XhoI/EcoRI-Adapters in die EcoRI-Stelle herstellen.

Das im nachfolgenden Beispiel 2 als Empfangsort für das DHFR-Gen verwendete Plasmid CaMV20-Bal 1 lässt sich aus dem CaMV-Stamm

CM4-184 gewinnen, indem man auf an sich bekannte Weise die Region ORF II durch Behandeln mit Bal 31 entfernt und in die Schnittstelle ein XhoI-Kupplungsfragment einführt.

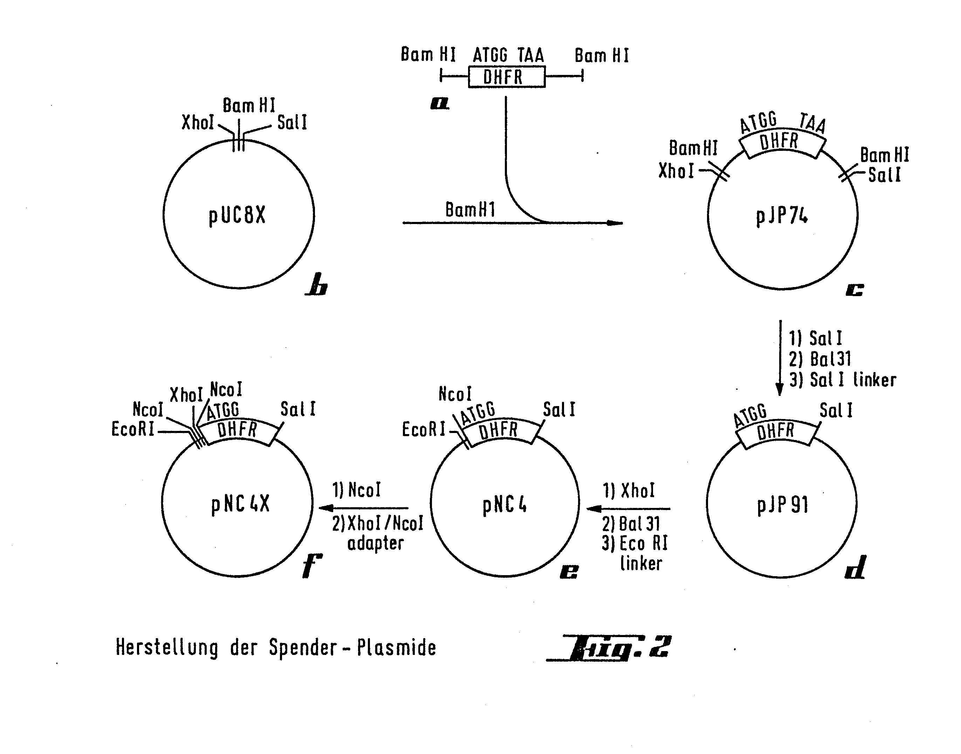

Beispiel 1: Ein Bam HI-Fragment (Figur 2a), welches das vollständige DHFR-Gen enthält, wird isoliert und in das Plasmid pUC8X (Figur 2b) eingespleist, in welches vor der Einführung des Gens eine XhoI-Restriktionsstelle für den nachfolgenden Transfer des DHFR-Gens eingeführt worden ist. Man erhält so das Plasmid .pJP74 (Figur 2c).

Beispiel 2:

a) Das Plasmid pJP74 wird mit dem Enzym SalI in die lineare Form übergeführt. Um die DNS-Sequenzen, welche den Strukturteil des DHFR-Gens flankieren, so weit wie möglich zu verkürzen, wird das Plasmid am 3'-Ende des DHFR-Gens mit der Nuklease Bal 31 behandelt. Anschliessend wird ein Kupplungsfragment SalI am 3'-Ende des DHFR-Gens eingefügt. Plasmide, welche Deletionen aufweisen und bei Escherichia coli noch eine Resistenz gegen das Antibioticum Trimethoprim (2,4-Diamino-5-(3,4,5-trimethoxybenzyl)-pyrimidin) bewirken, werden mittels DNS-Sequenzanalyse analysiert. Eines dieser Plasmide, das Plasmid pJP91 (Figur 2d), welches das Stop-Codon nebst 5 Basenpaaren der kodierenden DNS nicht mehr enthält, wird für die weitere Behandlung ausgewählt. Die DNS-Sequenz, welche das 5'-Ende des DHFR-Gens flankiert, wird in entsprechender Weise wie für das 3'-Ende vorstehend beschrieben, durch Behandlung mit der Nuklease Bal 31 verkürzt (Figur 2). Das XhoI/SalI-Fragment des Plasmids pJP91 wird isoliert und dient zum Einbau in die Virus-DNS.

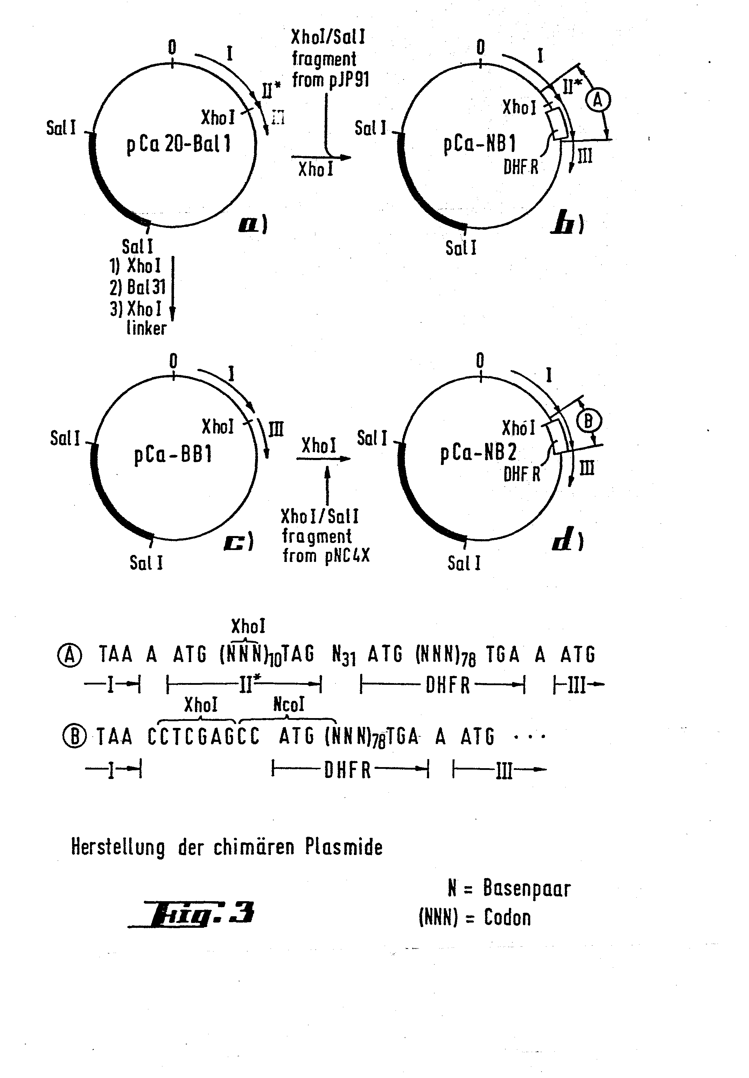

b) Als Empfangsort beim Cauliflower-Mosaik-Virus für das DHFR-Gen wird das Plasmid CaMV20-Bal 1 (Figur 3a) verwendet, bei welchem es sich um ein chimäres CaMV-Plasmid handelt, welches von der natürlichen Deletionsmutante CM4-184 abstammt und in das ein XhoI-Kupplungsfragment eingeführt worden ist. In diesem Plasmid fehlt mit Ausnahme der ersten fünf Codons und dem Translations-Stopsignal TGA die gesamte Region II. Es enthält eine einzige XhoI-Schnittstelle, welche sich unmittelbar vor dem Stop-Codon in der Region II
befindet.

Das isolierte XhoI/SalI-Fragment des Plasmids pJP91 wird am XhoI-Ort
des Plasmids pCaMV20-Bal 1 eingefügt und man erhält so das Plasmid
pCaMV-NB1 (Figur 3b), welches das DHFR-Gen im richtigen Orientierungssinn eingebaut enthält. Hierbei wird die Expression (Transkription und Translation) von Signalsequenzen der ursprünglichen
CaMV-DNS gesteuert.

Beispiel 3:

a) Das Plasmid pJP91 wird mit dem Enzym XhoI in die lineare Form
übergeführt und mit der Nuklease Bal 31 behandelt, um die das
5'-Ende flankierende Sequenz zu entfernen. Durch Einsetzen einer
EcoRI-Kupplungssequenz entsteht an den Bruchstellen, die mit dem
Startcodon ATGG beginnen, die Restriktionsstelle für NcoI (CCATGG).
Plasmide, welche diese Schnittstelle besitzen, werden für die
weitere Behandlung ausgewählt (Plasmid pNC4, Figur 2e). Bei Verwendung eines NcoI/XhoI-Kupplungsfragments an der NcoI-Stelle wird
so die XhoI-Stelle eingeführt. Man erhält so das Plasmid pNC4X
(Figur 2f).

b) Als Empfangsort beim Cauliflower Mosaik-Virus für das DHFR-Gen
wird das Plasmid pCaMV20-Bal 1 (Figur 3a) verwendet, aus dem die
Region ORF II vollständig entfernt worden ist. In das so erhaltene
Plasmid pCaMV-BB1 (Figur 3c) wird das isolierte XhoI/SalI-Fragment
des Plasmids pNC4X eingefügt. Der so entstandene Vektor, das Plasmid
pCaMV-NB2 (Figur 3d), weist zwischen dem Stop-Codon des Gens I und
dem Start-Codon ATG des DHFR-Gens nur 9 Basenpaare auf, während die
Anordnung am 3'-Ende dieselbe ist wie beim Plasmid pCaMV-NB1,
nämlich 1 Basenpaar (Figur 3).

Beispiel 4: Pflanzen der Spezies Brassica rapa werden mit Viren,
welche eines der Plasmide pCaMV-NB1 oder pCaMV-NB2 enthalten,
inokuliert. Blattstreifen von so infizierten Pflanzen und von
nicht-infizierten Pflanzen werden in üblichen festen und flüssigen Kulturmedien, welche Methotrexat in verschiedenen Konzentrationen
enthalten, kultiviert. 4 Tage nach dem Verbringen der Blattstreifen
in die Kulturmedien zeigen sich an den Blattstreifen deutliche
Unterschiede. Ab einer Konzentration von Methotrexat von 0,01 µM
zeigen nur diejenigen Blattstreifen ein normales Aussehen, welche
mit pCaMV-NB1- oder pCaMV-NB2-Viren infiziert worden sind. Die
nicht-infizierten Blattstreifen und solche, die mit pCaMV20-Bal 1-
oder pCaMV-BB1-Viren infiziert worden sind, zeigen nekrotische
Bereiche und Veränderungen in der Farbe. 6 Tage nach dem Einbringen
der Blattstreifen in die Kulturmedien ergibt die mikroskopische
Untersuchung, dass nur bei Geweben solcher Pflanzen, die mit
pCaMV-NB1- oder pCaMV-NB2-Viren infiziert worden waren, zelluläre
Expansion und Proliferation feststellbar sind.

Beispiel 5: Blattstreifen, welche wie im vorstehenden Beispiel 4
beschrieben, in Methotrexat-haltigen Kulturmedien kultiviert worden
sind, werden nach einer gewissen Zeitspanne in ein entsprechendes
Kulturmedium ohne Methotrexat übergeführt. Bei einer Verweilzeit von
24 Stunden in einem Kulturmedium mit Konzentrationen von 0,01 bis
0,2 µM Methoxtrexat sterben die Kontrollgewebe ab. Nach Ueberführung
in Kulturmedien ohne Methotrexat bilden die Gewebe, die mit pCaMV-
NB1- oder pCaMV-NB2-Viren infiziert worden waren, Kalli aus.

Beispiel 6: Je zwei Pflanzen der Spezies Brassica rapa werden mit
pCaMV-NB1- oder pCaMV20-Bal 1-Viren infiziert und mit 15 ml einer
Lösung von 50 µg Methotrexat/ml besprüht. Eine Woche nach der
Behandlung mit Methotrexat sind die mit pCaMV20-Bal 1-Viren infizierten Pflanzen fast gänzlich abgestorben, während die mit pCaMV-
NB1-Viren infizierten Pflanzen keine Schäden zeigen.

Patentansprüche:

1. Verfahren zur Herstellung einer chimären DNS, welche aus Virus-
DNS von einem Pflanzenvirus und einem in pflanzlichem Material zur
Expression gelangenden Fremdgen besteht, dadurch gekennzeichnet,
dass man in die DNS eines Pflanzenvirus ein in pflanzlichem Material
zur Expression gelangendes Fremdgen einsetzt.

2. Verfahren nach Anspruch 1, dadurch gekennzeichnet, dass man eine
oder mehrere Virus-DNS-Regionen, welche für die Vermehrung des die
chimäre DNS enthaltenden Virus nicht erforderlich sind, entfernt und
das Fremdgen an einem der Deletionsorte einsetzt, wobei es eine oder
mehrere der entfernten Virus-DNS-Regionen ersetzt.

3. Verfahren nach Anspruch 1, dadurch gekennzeichnet, dass man das
Fremdgen in die Virus-DNS mittels Restriktionsenzymen, für welche in
der Virus-DNS nur eine einzige Restriktionsschnittstelle vorliegt,
so einsetzt, dass die Erkennungssequenzen dieser Restriktionsenzyme
das einzusetzende Fremdgen an beiden Enden flankieren.

4. Verfahren nach Anspruch 1, dadurch gekennzeichnet, dass man den
Abstand zwischen dem Stopsignal des unmittelbar vor dem eingesetzten
Fremdgen liegenden Virusgens und dem Startsignal des eingesetzten
Fremdgens so kurz wie möglich hält.

5. Verfahren nach Anspruch 1, dadurch gekennzeichnet, dass man die
flankierenden Sequenzen des einzusetzenden Gens mit einer Nuklease
entfernt.

6. Verfahren nach Anspruch 1, dadurch gekennzeichnet, dass man den
Abstand zwischen dem Stopsignal des eingesetzten Fremdgens und dem
Startsignal des dem Fremdgen unmittelar nachfolgenden Virusgens so
kurz wie möglich hält.

7. Verfahren nach Anspruch 1, dadurch gekennzeichnet, dass man in die Virus-DNS ein Fremdgen von einer Grösse einführt, welches die Grösse des entfernten Virus-DNS-Fragments oder die Grösse, welche der Summe aller entfernten Virus-DNS-Fragmente entspricht, nicht wesentlich überschreitet.

8. Verfahren nach Anspruch 1, dadurch gekennzeichnet, dass man in die Virus-DNS eine oder mehrere Kodierungsregionen unter Beibehaltung des Leserasters der Virus-DNS einsetzt.

9. Verfahren nach Anspruch 1, dadurch gekennzeichnet, dass das einzusetzende Fremdgen keine homologen Enden aufweist und im richtigen Orientierungssinn eingefügt ist.

10. Verfahren nach Anspruch 1, dadurch gekennzeichnet, dass man als Pflanzenvirus ein DNS-Virus oder eine DNS-Kopie einer Virus-RNS verwendet.

11. Verfahren nach Anspruch 10, dadurch gekennzeichnet, dass man ein Caulimovirus verwendet.

12. Verfahren nach Anspruch 11, dadurch gekennzeichnet, dass man originale oder recombinante Stämme des Cauliflower-Mosaik-Virus verwendet.

13. Verfahren nach Anspruch 12, dadurch gekennzeichnet, dass man die zu entfernende DNS-Region aus einem oder mehreren der Bereiche ORF II, ORF VII oder der intergenomen Region der DNS von Cauliflower-Mosaik-Virus wählt.

14. Verfahren nach Anspruch 13, dadurch gekennzeichnet, dass man die Virus-DNS-Region ORF II aus der Virus-DNS entfernt und durch ein Fremdgen ersetzt und zwischen der tRNS-Bindungsstelle der Virus-DNS und der ersten, in Leserichtung folgenden ORF-Region nicht mehr als 50 bis 70 Basenpaare beibehält und die nachfolgenden ORF-Regionen in möglichst kurzen Abständen folgen.

15. Verfahren nach Anspruch 11, dadurch gekennzeichnet, dass man als Fremdgen das DHFR-Gen einsetzt.

16. Verfahren nach Anspruch 15, dadurch gekennzeichnet, dass man die flankierenden Sequenzen des DHFR-Gens mit der Nuklease Bal 31 möglichst vollständing entfernt und am 5'-Ende einer Kodiersequenz, bei welcher auf das Startsignal ATG ein weiteres G folgt, eine NcoI-Restriktionsstelle (CCATGG) einführt.

17. Verfahren nach Anspruch 12, dadurch gekennzeichnet, dass man die NcoI-Sequenz CCATGG als kennzeichnendes Merkmal für die Selektionierung des gewünschten Moleküls und zum Aufbau des chimären CaMV-Vektors verwendet.

18. Chimäre DNS, dadurch gekennzeichnet, dass sie aus der DNS von Cauliflower-Mosaik-Virus und einem Fremdgen besteht, welches an die Stelle der entfernten Region ORF II tritt.

19. Chimäre DNS nach Anspruch 18, dadurch gekennzeichnet, dass sie als Fremdgen das DHFR-Gen enthält.

20. Cauliflower-Mosaik-Virus, welches chimäre DNS gemäss Anspruch 19 enthält.

21. Pflanzliches Material, welches mit Cauliflower-Mosaik-Virus gemäss Anspruch 20 infiziert ist.

22. Das Plasmid pJP74.

23. Das Plasmid pJP91.

24. Das Plasmid pNC4.

25. Das Plasmid pNC4X.

26. Das Plasmid pCaMV-NB1.

27. Das Plasmid pCaMV-NB2.

28. Das Plasmid pCaMV-BB1.

29. Verwendung einer chimären DNS nach Anspruch 1 zur Infizierung von pflanzlichem Material, wobei man ein die chimäre DNS enthaltendes Pflanzenvirus als Vektor einsetzt.

30. Verwendung gemäss Anspruch 29 einer chimären DNS, welche aus der DNS von Cauliflower-Mosaik-Virus und dem DHFR-Gen, welches an die Stelle der aus der Virus-DNS entfernten Region ORF II tritt, besteht, wobei man das die chimäre DNS enthaltende Cauliflower-Mosaik-Virus als Vektor einsetzt.

FO 7.5 SES/eg*

*Fig.1*

Karte von CaMV

Herstellung der Spender-Plasmide          *Fig. 2*

0149430

a) pCa 20-Bal 1 — SalI, SalI, XhoI, 0, I, II*, III

XhoI/SalI fragment from pJP91

b) pCa-NB1 — SalI, SalI, XhoI, 0, I, II*, DHFR, III, Ⓐ

SalI
1) XhoI
2) Bal 31
3) XhoI linker

c) pCa-BB1 — SalI, SalI, XhoI, 0, I, III

XhoI/SalI fragment from pNC4X

d) pCa-NB2 — SalI, SalI, XhoI, 0, I, DHFR, III, Ⓑ

XhoI
Ⓐ TAA A ATG (NNN)$_{10}$ TAG N$_{31}$ ATG (NNN)$_{78}$ TGA A ATG
—I—| |———II*———| |———DHFR———| |—III—→

XhoI   NcoI
Ⓑ TAA CCTCGAGCC ATG (NNN)$_{78}$ TGA A ATG · · ·
—I—| |———DHFR———| |———III———→

Herstellung der chimären Plasmide

Fig. 3

N = Basenpaar
(NNN) = Codon

Europäisches
Patentamt

## EUROPÄISCHER RECHERCHENBERICHT

| | EINSCHLÄGIGE DOKUMENTE | | EP 84810636.5 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | <u>EP - A2 - 0 067 553</u> (NATIONAL RESEARCH COUNCIL OF CANADA) <br> * Patentansprüche 1,21 * | 1,29 | C 12 N 15/00 <br> C 12 N 7/00 |
| A | PROCEEDINGS OF THE NATIONAL ACADEMY OF SCIENCES OF THE UNITED STATES OF AMERICA, Vol. 80, No. 15, August 1983 (Baltimore, USA) <br> R.T. FRALEY et al. "Expression of bacterial genes in plant cells" Seiten 4803-4807 <br> * Ganzer Artikel * | 1 | |

| RECHERCHIERTE SACHGEBIETE (Int. Cl.4) |
|---|
| C 12 N |

Der vorliegende Recherchenbericht wurde für alle Patentansprüche erstellt.

| Recherchenort | Abschlußdatum der Recherche | Prüfer |
|---|---|---|
| WIEN | 27-03-1985 | WOLF |